# EUROPEAN PATENT SPECIFICATION

(11) **EP 2 957 891 B1**
(45) Date of publication and mention of the grant of the patent: **07.03.2018**
(21) Application number: 14751714.8
(22) Date of filing: 20.01.2014
(51) Int. Cl.: G01N 21/31, G01N 21/15, G01N 21/71, G01N 21/74, G01N 33/00, G01J 3/28

(54) **MERCURY MONITOR**
QUECKSILBERMONITOR
MONITEUR AU MERCURE

(30) Priority: 15.02.2013 RU 2013107775
(43) Date of publication of application: 23.12.2015
(73) Proprietor: Stroganov, Alexander Anatolyevich, St.Petersburg 196135 (RU); Sholupov, Sergei Evgenievich, St. Petersburg, 197341 (RU); Pitirimov, Pavel Vladimirovich, St.Petersburg 195220 (RU)
(72) Inventor: Stroganov, Alexander Anatolyevich, St.Petersburg 196135 (RU); Sholupov, Sergei Evgenievich, St. Petersburg, 197341 (RU); Pitirimov, Pavel Vladimirovich, St.Petersburg 195220 (RU)
(74) Representative: Sloboshanin, Sergej
(86) International application number: PCT/RU2014/000031
(87) International publication number: WO 2014/126507

(56) References cited:
- EP-A1- 1 469 299
- JP-A- 2011 127 988
- RU-C1- 2 110 060
- US-A- 5 679 957
- US-A- 5 939 648
- US-A1- 2012 161 022
- 'MERCEM300Z. Mercury Analyzer.' OPERATION MAINTENANCE 2011, pages 12 - 15

## Description

The claimed invention refers to analytical systems of automatic measurement of mercury concentration and can be used to monitor industrial and sewage water and combustion gases.

The mercury analyzer PA-2 Mercury Process Analyzer by Mercury Instruments, Germany [1] designed for continuous measurement of concentration of mercury in industrial sewage waters of the enterprises dedicated to burning of waste, thermal power plants, treatment facilities, etc., is known currently. The mercury monitor contains: the sample preparation module where the preliminary oxidation of a sample with corresponding reagent takes place, the reduction module where mercury is reduced to atomic state at addition of a reducer; the gas exchange unit where elemental mercury is released from the liquid sample and comes into gas carrier, and the analytical cell where gas-carrier delivers elemental mercury and where the amount of released mercury is defined with atomic - absorption method.

The drawbacks of the analogue are: need to perform preliminary work for choice of the method of oxidation of the sample (in dependence on the sample matrix and forms of presence of mercury in the sample), high requirements to purity of the reactants used both at sample preparation, and at reducing of mercury to atomic state, as well as short period of monitor unattended operation that is caused by precipitation of reducer and hardness salts from reducing solution and from the sample respectively. Besides, it requires frequent filling up to compensate for spent reactants.

The device to monitor mercury emissions [2] is known containing input unit for gas sample to be analyzed, the thermal atomizer where all mercury compounds dissociate with formation of elemental mercury, the analytical cell capable of being heated that decreases the rate of oxidation of elemental mercury with dissociation products considerably and matrix components, the atomic - absorption spectrometer allowing to measure elemental mercury and decrease influence of matrix significantly.

The drawbacks of the analogue are: short period of monitor unattended operation caused by pollution of analytical cell windows, impossibility to use the device to monitor industrial waters because of precipitation of high concentration dissolved salts, containing in them, on walls of the input sample unit and the thermal atomizer leading also to blocking gas channels.

US 2012/161022 discloses an apparatus and method operable for the continuous monitoring of a gas stream including an optical sensor operable to monitor and/or measure Hg concentrations in a flue gas by calculating the absorbance of the ultraviolet light thereby at a range of wavelength 253.7 nm+/-0.05 nm. The apparatus therefore provides as a spectrally narrow UV light source, a mercury lamp. The spectrally broad UV light source includes a UV LED. A coupler is provided to mix the narrow and broad UV light energy which is propagated through the gas stream. The invention recognizes that measurement of radiation absorption at the 254 nm+/-1.5 nm range will result in not only from Hg0, but also from the SO2 component in the flue gas.

EP 1 469 299 refers to a measuring gas cuvette which comprises 2 opposing windows for passage of radiation and is positioned in a horizontal working position, with a gas inlet for a measuring gas arranged between 2 gas outlets, adjacent respective windows, so that the measuring gas is fed into the measuring gas cuvette at its center and divided into 2 partial streams, flowing in opposite directions to the gas outlets.

JP 2011 127988 discloses a gas measurement cell which includes a main pipe opened at both ends and coupled to a flow path for flowing an exhaust gas at both ends, an inlet pipe coupled to the main pipe and formed with a window for transmitting a light at the end opposite to the side coupled to the main pipe, an outlet pipe coupled to the main pipe, and formed with a window for transmitting a light at the end opposite to the side coupled to the main pipe, and a first purge gas supply pipe coupled to the inlet pipe. The inlet pipe has an opening area at the end on the side coupled to the main pipe larger than an opening area at the end of the window.

The mercury monitor of combustion gases MERCEM300Z Mercury Analyzer, of the firm Sick, Germany [3] is the closest technical-wise consisting of the sampling probe, the gas line, the sample input unit, the thermal atomizer, the analytical cell capable of being heated, the atomic - absorption spectrometer and the sucking out pump. Combustion gas is taken with sampling probe and is transported to the input part of the monitor. Further gas passes into the thermal atomizer where all mercury irrespective of a form of its presence in combustion gas is transformed into elemental form, and comes to the analytical cell where mercury concentration is defined by means of the atomic - absorption spectrometer. The sucking out pump manufactured in the form of an ejector is attached to the analytical cell exit. The thermal atomizer and the analytical cell temperature is 1000 * C.

The drawbacks of the prototype are: short period of monitor unattended operation caused by pollution of analytical cell windows. Combustion gas has quite complex composition - various gases (CO2, SO2, NO, NOx, water vapors, HCl, HF etc.) which are released and formed at mineral fuel burning, as well as smoke particles. Interaction of aggressive gases with smoke particles and elements of the sampling system leads to formation of volatile compounds which precipitate on the windows of the analytical cell because gas temperature is about 1000 * C, while temperature of the windows on the outer side is much lower. Pollution of the windows leads to considerable decrease in intensity of the probing radiation of the atomic-absorption spectrometer and, as a result, to deterioration of analytical characteristics and even to impossibility to carry out measurements. For the same reasons, it is impossible to use the prototype to define content of mercury in industrial waters of various enterprises - water contains high percentage concentrations of chlorides and sulfates of metals (hardness salts) and evaporation and atomization lead to formation of vapors of these salts which would precipitate on the analytical cell windows.

The task of the proposed invention is improvement of consumer characteristics of the monitor, increase in unattended operating time of the monitor and ensuring long functioning of the monitor.
1. The set task is achieved by the mercury monitor including the input sample unit, the thermal atomizer, the analytical cell capable of being heated, the gas collector unit, and the sucking out pump, at that the analytical cell contains two windows being transparent for resonant radiation of mercury, through one of which (at least) it is optically coupled with the atomic-absorption spectrometer; at least, one input gas port is located in its central part and, at least, two output gas ports, each of which is located between input gas port and the corresponding window, wherein the sample input unit is coupled with injecting pump capable of introducing the sample analyzed into the thermal atomizer, and the analytical cell has openings located in its both ends between the window and the nearest output gas port through which possibility of gas supply is realized.

The essence of the invention is that protective air stream is created between the window of analytical cell and the gas analyzed preventing direct contact of the hot gas to be analyzed arriving in the analytical cell with cold surface of the window. Owing to this fact, vapors of highly volatile compounds, present in the gas to be analyzed, aren't condensed on the windows of the analytical cell, and transmission coefficient of the windows for probing radiation of the atomic - absorption analyzer remains in the working range for a long time.

The mercury monitor also contains the nebulizer with its holder between the internal wall of which and the nebulizer itself, a cavity that is connected with internal cavity of the thermal atomizer, while the holder itself contains a port connecting the cavity created with carrier-gas supply means. The nebulizer contains a spraying nozzle, the liquid input port and the gas input port which is connected by means of gas-liquid communication with the gas-carrier supply means. At that the carrier-gas supply means contain the mixer with three ports; the first port is connected to the gas-carrier source, the second port is connected to the water supply means, and the third port of the mixer is connected to the second port of the nebulizer.

Besides, the gas collector unit is connected with output ports of the analytical cell by means of gas-liquid communication and includes the gas-liquid separator and the liquid collection reservoir.

At last, the analytical cell is optically connected with the spectrometer - through the first window, and with the retroreflector - through the second window, the retroreflector being assigned the way that probing radiation of the spectrometer having passed through the first window to the second one, should return back through the second window to the spectrometer through the first window.

Use of the nebulizer in the claimed mercury monitor allows to eliminate the mechanical water sample supply means to the atomizer that allows to increase reliability of the supplying device, as well as to input a sample in the form of aerosol that reduces amount of salts precipitating on the atomizer wall. Water supply into the nebulizer compressed air canal allows to decrease amount of the salts precipitating directly in its nozzle. Installation of the gas-liquid separator after the analytical cell allows to remove water vapors from output gas stream and thereby to eliminate condensation of water in the sucking out pump and to provide its regular operating mode. Use of optical scheme at which radiation passing through the analytical cell comes on a retroreflector, returns into the analytical cell and then goes to a photodetector, allows to double sensitivity of the analysis at the same linear sizes of analytical cell. Besides, such scheme provides compactness of spectrometer construction that increases stability of work of the entire spectrometer.

The essence of the claimed invention is illustrated by drawings:
Fig.1. Illustration of the mercury monitor.
Fig.2 Illustration of the input unit with the nebulizer and the gas supply means.
Fig.3 Illustration of the gas collector unit.
Fig.4 Scheme of the analytical cell windows' protection.
Fig.5 Graph of sensitivity dependence on flow rate of the sucking out pump.
Fig.6 Photograph of model of spraying water aerosol in the thermal atomizer.
Fig.7 Photograph of the windows of the analytical cell with protective air stream after 14 days of operation (A) and in 8 hours operation without it (B).

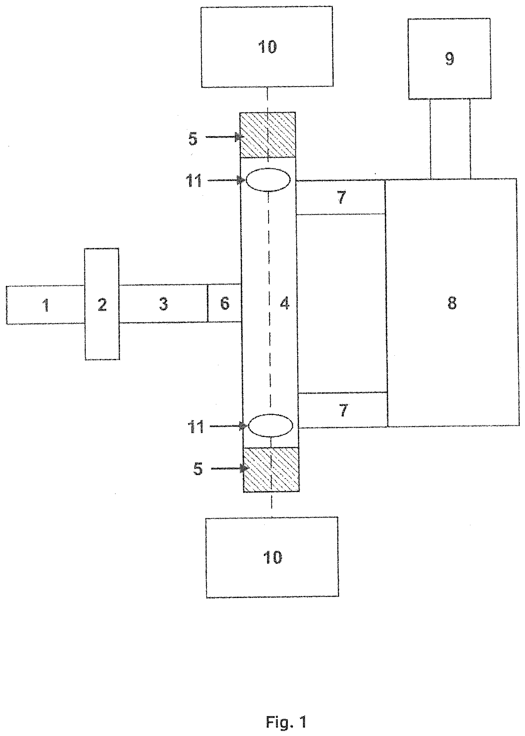

The mercury monitor, whose flow chart is presented in Fig.1, contains the unit of injecting pump 1, the sample input unit 2, the thermal atomizer 3, the analytical cell 4 with the windows 5 and with input 6 and output 7 gas ports, the gas collector unit 8, the sucking out pump 9 (hereinafter referred to as, the return pump), and also the atomic - absorption spectrometer 10. The case of the analytical cell has openings 11 between windows and the output ports.

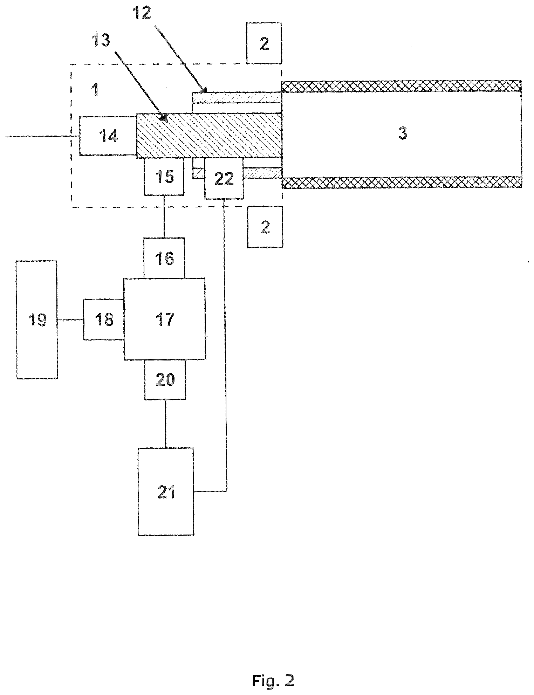

The unit of the injecting pump 1 can be performed as follows (Fig. 2). The nebulizer 13 is installed in the nebulizer holder 12 in such a way that its nozzle is directed towards internal part of the thermal atomizer 3, and all assembly is installed in the sample input unit 2. The liquid port of the nebulizer 13 is connected to the switching liquid tap (is not shown in the figure), which alternatively connects the specified port of the nebulizer with distilled water reservoirs, standard solution, and the sample to be analyzed. The gas port of the nebulizer 15 is connected to the first port 16 of the mixer 17, whose second port 18 is connected to water supply means 19, and its third port 20 is connected to carrier-gas supply means. 21. Carrier-gas is supplied also to the cavity between the nebulizer and its holder through the holder port 22.

When determining the content of mercury in combustion gases, the unit of the injecting pump 1 can be made in the form of the diaphragm pump supplying analyzed gas from the sampling line (is not shown in the figure) directly to the thermal atomizer 3.

The thermal atomizer 3 can be performed as a quartz tube whose one end is hermetically attached to sample input unit 2, and the second end is hermetically attached to the input port 6 of the analytical cell 4. And the quartz tube is installed coaxially with the nebulizer 13 and its holder 12, and its internal diameter isn't less, than the internal diameter of the nebulizer holder 12. The heater maintaining temperature inside the quartz tube in the range of 600 - 700 * C is installed on the outer side of the quartz tube. The entire thermal atomizer is placed into the metal protection enclosure.

The analytical cell 4 can be made in the form of the cylinder with the input port 6 hermetically welded to the middle part and two framed windows 5 are installed on its butt ends. The output ports 7 are installed between the windows 5 and the input port 6 on two sides closer to the windows. To form protective air stream, the openings 11 are made between the windows 5 and the output ports 7. The heaters maintaining temperature of the gas to be analyzed in the range of 600 - 750 * C are installed in the internal part of the cell.

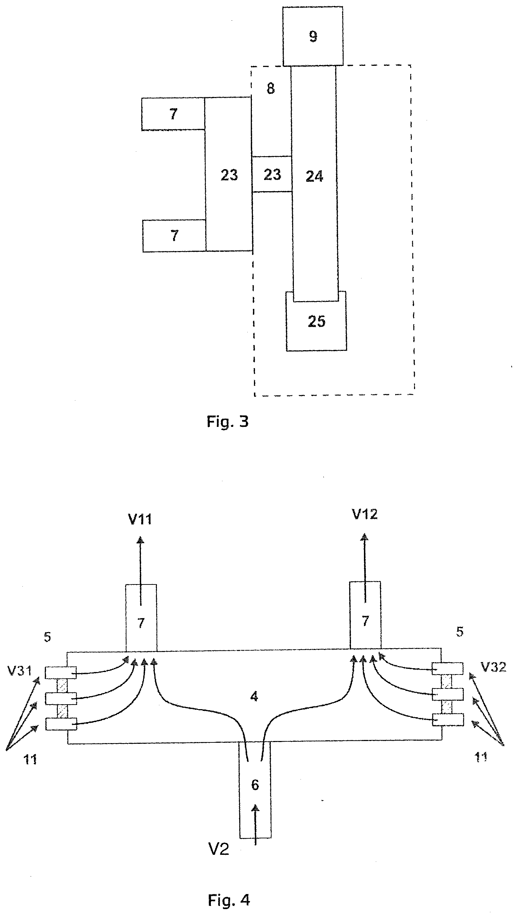

The gas collector unit 8 can be made as follows (Fig.3). The output ports 7 of the analytical cell 4 are connected by means of steam-gas communications 23 to the gas-liquid separator 24 which can be made in the form of the return refrigerator in whose external condenser jacket the cooling water flows, and steam-gas mix comes into its internal part. One end of the separator 24 is connected to the liquid collection reservoir 25 where water comes after cooling steam-gas mix. The second end of the separator 24 is connected to the sucking out pump 9 performed as a diaphragm pump.

The atomic - absorption spectrometer 10 can be made as the atomic - absorption analyzer of mercury with the direct Zeeman effect [4] which is characterized by high selectivity of measurements.

Let us consider operation of the mercury monitor on the example of determination of total content of mercury in technological water of a thermal power plant. Specifics of technological water as object of analysis, is that it contains the dissolved high concentration hardness salts (1 - 5%). The water to be analyzed comes into the tank which is connected to the input of the switching liquid tap. Other inputs of the valve are connected to the distilled water reservoir and with standard solution, necessary to carry out blank measurement and calibration of the monitor. The output of the switching liquid tap is connected to the liquid port of the nebulizer. The source of compressed air is connected to the gas port of the nebulizer. Compressed air purified of dust and oil vapors, for example, by means of the dust and oil filter, passing through the nebulizer, creates evacuation in the region of the gas nozzle (Venturi's effect) that leads to suction of liquid from the liquid channel of the nebulizer and its coming in the gas nozzle. Slowly incoming liquid is affected in the gas nozzle by fast air stream that leads to formation of water aerosol which comes further into the thermal atomizer. In the thermal atomizer whose temperature is in the range of 600 - 700*C, water evaporates from aerosol particles and all mercury containing in them converts into atomic form at this temperature. At evaporation of water the small solid particles of salts (salt aerosol) are formed and present is suspension in gas-carrier. Further all formed components are transported by gas-carrier to the unit of analytical cell through its input port. At the same time, the atmospheric air comes into the analytical cell through openings in the windows preventing direct contact of the gas analyzed with the windows' surface. Steam-gas mix comes from the output ports of the analytical cell through the gas tee into the gas - liquid separator, made as the return refrigerator in whose external cooling jacket the cooling water flows. The second end of the gas tee is connected with the pipeline to the liquid collection reservoir in which the water condensed in the return refrigerator is collected. The pipeline is installed in such a way that its second end in the liquid collection reservoir is always below water level (at the monitor start, an additional amount of water is filled in this reservoir), thus, performing function of a water lock for gas part of the scheme. The second end of the return refrigerator is connected to the return pump, inducing sucking-out gas stream after the analytical cell.
At formation of water aerosol inside of the nebulizer, the part of formed aerosol precipitates on the internal wall of the gas nozzle. As the water analyzed contains high concentration of hardness salts, evaporation of water from precipitated aerosol leads to accumulation of salts on internal surface of the gas nozzle that causes change of nozzle geometry and fast contamination of the nebulizer. To eliminate contamination of the nebulizer an additional amount of distilled water is introduced into canal of compressed air and continuously washes out the nozzle and removes precipitated salts from it.

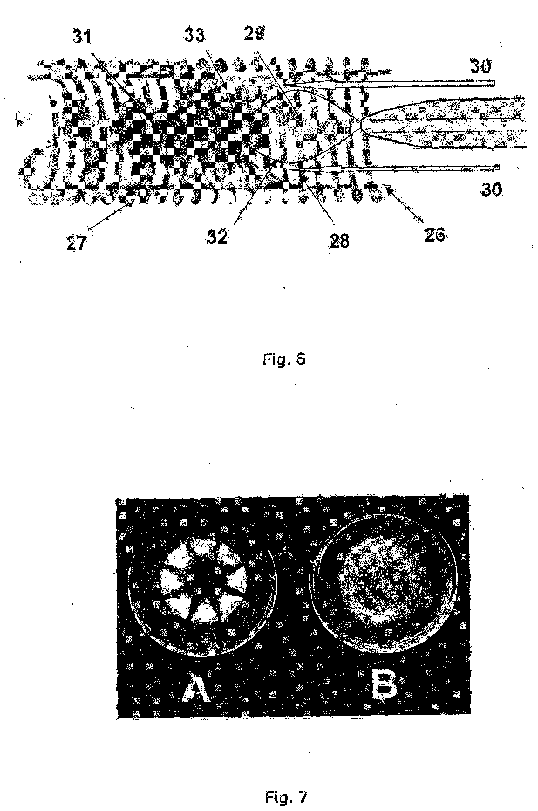

At injection of water aerosol into the thermal atomizer (Fig.6) the part of the aerosol evaporates directly in the carrier-gas, and the part (without full evaporation of water) succeeds to reach heated walls of the atomizer - the quartz tube 26 placed in the heater 27 that is determined by 28 water aerosol's 29 spraying finite angle. To increase the time spent by water aerosol in gas-carrier an air stream 30 is additionally injected between the nebulizer and its holder. Further this additional air stream extends along walls of the thermal atomizer and thereby retains the main stream of aerosol in axial zone of the atomizer where evaporation of water from an aerosol particle with formation of salt aerosol 31 actually takes place. Increase in water aerosol 32 motion trajectory leads to an increase in share of evaporated water aerosol in carrier-gas, a decrease in precipitation rate of salt 33 on the wall of the thermal atomizer and thereby - an increase in operating life of the unattended thermal atomizer.

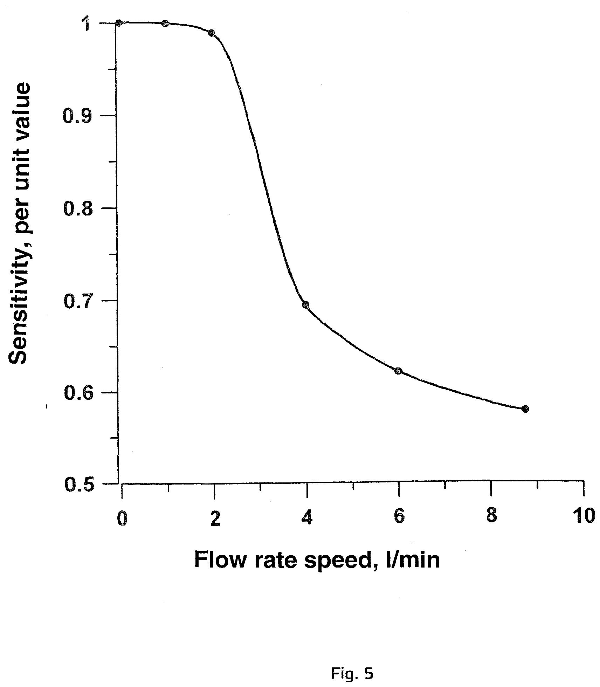

In process of transportation of salt aerosol inside the atomizer and in the heated cell (the analytical cell temperature is 650 - 750*C) salt compounds partially evaporate from aerosol particle surface and pass into carrier-gas in the form of vapor. Similarly, the salt compounds precipitated on atomizer surface, when heated, partially evaporate and pass into carrier-gas. At last, at interaction of salt particles with surface of the heaters of the analytical cell material of heating spirals partially evaporates, and its vapors pass into carrier-gas. To eliminate effect of precipitation of vapors of highly volatile compounds from carrier- gas on surface of the windows of the analytical cell, it was designed as having windows blown with clean air, and having no direct contact of the carrier-gas with cell windows. The protection scheme of analytical cell windows is presented in Fig.4. Gas is pumped out from the analytical cell through the output ports 7 with volume speed V1 = V11 + V12. The gas analyzed is supplied through the input port 6 with volume speed V2. The return pump 9 creates evacuation in the output ports and, respectively, in the analytical cell, the cell pressure being lower than atmospheric one. Ambient air stream in the analytical cell is created due to evacuation in the analytical cell. As openings are located in immediate proximity to the output ports, the created air stream enters openings and at once leaves through the output ports, without extending along an axis of the analytical cell. Speed of the return pump V1 is higher than supply speed of gas analyzed in the analytical cell V2, therefore, speed of protective air stream V3 = V31 + V32 will make the value V3 = V1 - V2. Dependence of measurement sensitivity on speed of the return pump V1 at constant rate of supply of gas analyzed V2 = 2 l/min is given in Fig.5. If pumping - out speed is less than supply speed (0-2 l/min), the gas analyzed occupies the entire cell, including the regions between the output ports and cell windows, sensitivity being maximal (under existing experiment conditions sensitivity is proportional to the effective length of the analyzed gas layer). With increase in pumping-out speed (2-4 l/min) the gas analyzed from region between windows and the output ports is replaced with atmospheric air, and, respectively, sensitivity of measurements drops. The further increase in pumping-out speed (4 - 9 l/min) leads to negligible change of sensitivity, i.e. increase of pumping speed leads only to increase in protective air stream at insignificant decrease of effective length of the layer of the gas analyzed. Concentration of mercury in air is to be such that air which has got into the analytical cell (together with mercury) shouldn't affect results of mercury measurement in a water sample. In our case, concentration of mercury shouldn't exceed value of 6 µg/m³ (at 1 hour stability at the level of 10%) that is virtually equal to threshold allowable concentration in working area (10 µg/m³). The developed construction of the analytical cell unit has been tested by means of analysis of a real water sample. The photos of windows with protective air stream (A) and without it (B) are shown on Fig. 7. The given figure allows to conclude that windows with protective stream remain operative (probing radiation of the atomic absorption spectrometer passes through the central part of windows) after 14 days while the windows without protective stream come into nonoperational state after 8 hours of operation.

Other example of work of the mercury monitor is determination of mercury content in combustion gases. Combustion gas has rather complex composition: smoke particles, water vapors, O2, CO2, NO, NO2, SO2, HCl, HF, Hg and its compounds and so on. In addition, temperature of gas in sampling point is 100 - 200 *C. The sampling probe is connected with the pumping unit - injecting pump heated with gas lines. As the injecting pump we propose to use the diaphragm pump with teflon coating of all elements contacting with gas stream. The output of the pressure pump is connected to the sample input unit after which gas analyzed comes into the thermal atomizer whose temperature is 800 - 950 * C. This temperature is enough to convert fixed mercury to elemental form, and also to considerably decrease the rate of oxidation of elemental mercury. Gas from the thermal atomizer is transported in the analytical cell heated to 850 - 950 * C, through its input gas port. To protect windows against precipitation of volatile compounds which are present in gas analyzed, the analytical cell has construction at which windows are blown with external clean air that prevents direct contact of the window with gas analyzed. The output gas port of the analytical cell is connected to the gas collector unit in which temperature of gas analyzed decreases to level, admissible for work of the sucking out pump performed as the diaphragm pump. As return pump is higher than flow of the pressure pump, it leads to appearance of differential stream through openings in region of the windows of the analytical cell, protecting windows from pollution.

Thus, the present invention allows to increase interval of mercury monitor unattended operation at least by 40 times.

### References

1. http://www.mercury-instruments.de/EN/products/downloads/Mercury-Instruments-PA-2-en.pdf
2. Patent US 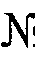 º 5,679,957.
3. http://www.sick.com MERCEM300Z Mercury Analyzer.
4. A. A. Ganeev, S.E. Shopulov, M. N. Slyadnev «Zeeman modulation polarization spectrometry as variance of atomic - absorption analysis: possibilities and constraints», JAC, 1996, v.51, no.8, p.855-864.

## Claims

1. A mercury monitor including a sample input unit (2), a thermal atomizer (3), an analytical cell (4) capable to be heated, a gas collector unit (8), and a sucking out pump (9);
wherein the analytical cell (4) comprises:
a body which is elongated along a longitudinal direction and includes a first butt end and a second butt end,
two windows (5), wherein a first window (5) is adjacent to the first butt end and a second window (5) is adjacent to the second butt end and the windows (5) are transparent for resonant radiation of mercury, wherein at least through one of the windows (5) the analytical cell (4) is optically coupled with an atomic absorption spectrometer (10),
at least one input gas port (6) located in central part of the analytical cell (4) and
at least two output gas ports (7), wherein a first output gas port (7) is installed between the first window (5) and the input gas port (6), closer to the first window (5), and a second output gas port (7) is installed between the second window (5) and the input gas port (6), closer to the second window(5),
wherein the output gas ports (7) are connected to a first side of the gas collector unit (8) and the sucking out pump (9) is connected to a second side of the gas collector unit (8) and is adapted to create a cell pressure being lower than atmospheric pressure, wherein the sample input unit (2) is coupled with an injecting pump (1) capable to introduce an analyzed sample into the thermal atomizer (3), wherein a first end of the thermal atomizer (3) is hermetically attached to the sample input unit (2) and a second end of the thermal atomizer (3) is hermetically attached to the input gas port (6) of the analytical cell (4); the analytical cell (4) has openings (11) through which air can come into an interior area of the analytical cell (4) to form a protective air stream between the windows (5) and hot gas to be analysed, wherein a first opening is located between the first window (5) and the first output gas port (7) and a second opening is located between the second window (5) and the second output gas port (7).

2. The monitor according to claim 1, wherein the injecting pump (1) contains a nebulizer (13) with a spraying nozzle, a liquid input port (14) and a gas input port (15) which is connected by gas liquid communication with means (21) of supply of carrier gas.

3. The monitor according to claim 2, wherein the means (21) of supply of carrier gas contain a mixer (17) with three ports wherein the first port (16) of the mixer (17) is connected to the gas input port (15) of the nebulizer (13), the second port (18) of the mixer is connected to means of water supply (19), and the third port (20) of the mixer (17) is connected to the means (21) of supply of carrier gas.

4. The monitor according to claim 2, wherein the sample input unit (2) contains a holder (12) of the nebulizer (13) wherein between an internal wall of the holder (12) and the nebulizer (13) a cavity is created, while the cavity is connected with an internal cavity of the thermal atomizer (3), and the holder (12) of the nebulizer (13) contains a port (22) connecting the created cavity to the means (21) of supply of carrier gas.

5. The monitor according to claim 1, wherein the gas collector unit (8) is connected with the output gas ports (7) of the analytical cell (4) by a gas liquid communication (23) and includes a gas-liquid separator (24) and a reservoir (25) for liquid collection.

6. The monitor according to claim 1, wherein the analytical cell (4) is optically coupled through the first window (5) with the atomic absorption spectrometer (10), and it is optically coupled through the second window (5) with a retroreflector assigned so that probing radiation of the atomic absorption spectrometer (10) which passed through the first window (5) in the second window (5) returns through the second window (5) back in the atomic absorption spectrometer (10) through the first window (5).

## Patentansprüche

1. Quecksilbermonitor, umfassend eine Probeneingangseinheit (2), einen thermischen Zerstäuber (3), eine aufheizbare Analysezelle (4), eine Gassammeleinheit (8) und eine Absaugpumpe (9);
wobei die Analysezelle (4) umfasst:
einen Körper, der in einer Längsrichtung langgestreckt ist und ein erstes stumpfes Ende und ein zweites stumpfes Ende aufweist;
zwei Fenster (5), wobei ein erstes Fenster (5) an das erste stumpfe Ende angrenzt und ein zweites Fenster (5) an das zweite stumpfe Ende angrenzt und die Fenster (5) für resonante Quecksilberstrahlung durchlässig sind, wobei die Analysezelle (4) wenigstens durch eines der Fenster (5) optisch mit einem Atom-Absorptions-Spektrometer (10) verbunden ist,
wenigstens eine Gaseintrittsöffnung (6), die in dem zentralen Teil der Analysezelle (4) angeordnet ist, und
wenigstens zwei Gasaustrittsöffnungen (7), wobei eine erste Gasaustrittsöffnung (7) zwischen dem ersten Fenster (5) und der Gaseintrittsöffnung (6), die näher bei dem ersten Fenster (5) ist, angeordnet ist,
und eine zweite Gasaustrittsöffnung (7) zwischen dem zweiten Fenster (5) und der Gaseintrittsöffnung (6), die näher bei dem zweiten Fenster (5) ist, angeordnet ist,
wobei die Gasaustrittsöffnungen (7) mit einer ersten Seite der Gassammeleinheit (8) verbunden sind und die Absaugpumpe (9) mit einer zweiten Seite der Gassammeleinheit (8) verbunden ist und einen Zelldruck erzeugen kann, der niedriger als der atmosphärische Druck ist,
wobei
die Probeneingangseinheit (2) mit einer Einspritzpumpe (1) verbunden ist, die eine analysierte Probe in den thermischen Zerstäuber (3) einführen kann, wobei ein erstes Ende des thermischen Zerstäubers (3) abdichtend an der Probeneingangseinheit (2) angebracht ist und ein zweites Ende des thermischen Zerstäubers (3) abdichtend an der Gaseintrittsöffnung (6) der Analysezelle (4) angebracht ist;
die Analysezelle (4) Öffnungen (11) aufweist, durch die Luft in einen Innenbereich der Analysezelle (4) gelangen kann, um einen schützenden Luftstrom zwischen den Fenstern (5) und dem zu analysierenden heißen Gas zu bilden,
wobei eine erste Öffnung zwischen dem ersten Fenster (5) und der ersten Gasaustrittsöffnung (7) angeordnet ist und eine zweite Öffnung zwischen dem zweiten Fenster (5) und der zweiten Gasaustrittsöffnung (7) angeordnet ist.

2. Monitor nach Anspruch 1, wobei die Einspritzpumpe (1) einen Vernebler (13) mit einer Sprühdüse, eine Flüssigkeitseintrittsöffnung (14) und eine Gaseintrittsöffnung (15) umfasst, die durch eine Gas-Flüssigkeits-Verbindung mit einem Mittel (21) zur Trägergaszufuhr verbunden ist.

3. Monitor nach Anspruch 2, wobei das Mittel (21) zur Trägergaszufuhr einen Mischer (17) mit drei Öffnungen umfasst, wobei die erste Öffnung (16) des Mischers (17) mit der Gaseintrittsöffnung (15) des Verneblers (13) verbunden ist, die zweite Öffnung (18) des Mischers mit einem Wasserzufuhrmittel (19) verbunden ist und die dritte Öffnung (20) des Mischers (17) mit dem Mittel (21) zur Trägergaszufuhr verbunden ist.

4. Monitor nach Anspruch 2, wobei die Probeneingangseinheit (2) eine Halterung (12) des Verneblers (13) umfasst, wobei zwischen einer Innenwand der Halterung (12) und dem Vernebler (13) ein Hohlraum ausgebildet ist, während der Hohlraum mit einem inneren Hohlraum des thermischen Zerstäubers (3) verbunden ist und die Halterung (12) des Verneblers (13) eine Öffnung (22) umfasst, die den ausgebildeten Hohlraum mit dem Mittel (21) zur Trägergaszufuhr verbindet.

5. Monitor nach Anspruch 1, wobei die Gassammeleinheit (8) mit den Gasaustrittsöffnungen (7) der Analysezelle (4) durch eine Gas-Flüssigkeits-Verbindung (23) verbunden ist und einen Gas-Flüssigkeits-Separator (24) sowie einen Behälter (25) zur Flüssigkeitssammlung aufweist.

6. Monitor nach Anspruch 1, wobei die Analysezelle (4) durch das erste Fenster (5) optisch mit dem Atom-Absorptions-Spektrometer (10) verbunden ist und durch das zweite Fenster (5) optisch mit einem Retro-Reflektor verbunden ist, der so angeordnet ist, dass Prüfstrahlung des Atom-Absorptions-Spektrometers (10), die durch das erste Fenster (5) in das zweite Fenster (5) gelangt ist, durch das zweite Fenster (5) in das Atom-Absorptions-Spektrometer (10) durch das erste Fenster (5) zurückkehrt.

## Revendications

1. Moniteur à mercure incluant une unité d'entrée d'échantillon (2), un atomiseur thermique (3), une cellule analytique (4) apte à être chauffée, une unité collectrice de gaz (8), et une pompe d'aspiration (9) ;
dans lequel la cellule analytique (4) comprend :
un corps qui est allongé le long d'une direction longitudinale et inclut une première extrémité d'aboutement et une deuxième extrémité d'aboutement,
deux fenêtres (5), dans lequel une première fenêtre (5) est adjacente à la première extrémité d'aboutement et une deuxième fenêtre (5) est adjacente à la deuxième extrémité d'aboutement et les fenêtres (5) sont transparentes pour un rayonnement résonant de mercure, dans lequel, au moins à travers l'une des fenêtres (5), la cellule analytique (4) est optiquement couplée à un spectromètre d'absorption atomique (10),
au moins un orifice de gaz d'entrée (6) situé dans la partie centrale de la cellule analytique (4) et
au moins deux orifices de gaz de sortie (7), dans lequel un premier orifice de gaz de sortie (7) est installé entre la première fenêtre (5) et l'orifice de gaz d'entrée (6), plus proche de la première fenêtre (5), et un deuxième orifice de gaz de sortie (7) est installé entre la deuxième fenêtre (5) et l'orifice de gaz d'entrée (6), plus proche de la deuxième fenêtre (5),
dans lequel les orifices de gaz de sortie (7) sont raccordés à un premier côté de l'unité collectrice de gaz (8) et la pompe d'aspiration (9) est raccordée à un deuxième côté de l'unité collectrice de gaz (8) et est adaptée à créer une pression de cellule qui est inférieure à la pression atmosphérique, dans lequel
l'unité d'entrée d'échantillon (2) est accouplée à une pompe d'injection (1) apte à introduire un échantillon analysé à l'intérieur de l'atomiseur thermique (3), dans lequel une première extrémité de l'atomiseur thermique (3) est hermétiquement fixée sur l'unité d'entrée d'échantillon (2) et une deuxième extrémité de l'atomiseur thermique (3) est hermétiquement fixée sur l'orifice de gaz d'entrée (6) de la cellule analytique (4) ;
la cellule analytique (4) a des ouvertures (11) à travers lesquelles de l'air peut pénétrer dans une zone intérieure de la cellule analytique (4) afin de former un courant d'air protecteur entre les fenêtres (5) et du gaz chaud à analyser, dans lequel une première ouverture est située entre la première fenêtre (5) et le premier orifice de gaz de sortie (7) et une deuxième ouverture est située entre la deuxième fenêtre (5) et le deuxième orifice de gaz de sortie (7).

2. Moniteur selon la revendication 1, dans lequel la pompe d'injection (1) contient un nébuliseur (13) avec une buse de pulvérisation, un orifice d'entrée de liquide (14) et un orifice d'entrée de gaz (15) qui est raccordé par communication gaz liquide à des moyens (21) d'apport de gaz porteur.

3. Moniteur selon la revendication 2, dans lequel les moyens (21) d'apport de gaz porteur contiennent un mélangeur (17) avec trois orifices dans lequel le premier orifice (16) du mélangeur (17) est raccordé à l'orifice d'entrée de gaz (15) du nébuliseur (13), le deuxième orifice (18) du mélangeur est raccordé à des moyens d'apport d'eau (19), et le troisième orifice (20) du mélangeur (17) est raccordé aux moyens (21) d'apport de gaz porteur.

4. Moniteur selon la revendication 2, dans lequel l'unité d'entrée d'échantillon (2) contient un élément de support (12) du nébuliseur (13) dans lequel, entre une paroi interne de l'élément de support (12) et le nébuliseur (13), une cavité est créée, tandis que la cavité est raccordée à une cavité interne de l'atomiseur thermique (3), et l'élément de support (12) du nébuliseur (13) contient un orifice (22) raccordant la cavité créée aux moyens (21) d'apport de gaz porteur.

5. Moniteur selon la revendication 1, dans lequel l'unité collectrice de gaz (8) est raccordée aux orifices de gaz de sortie (7) de la cellule analytique (4) par une communication gaz liquide (23) et inclut un séparateur gaz-liquide (24) et un réservoir (25) pour le recueil de liquide.

6. Moniteur selon la revendication 1, dans lequel la cellule analytique (4) est optiquement couplée à travers la première fenêtre (5) au spectromètre d'absorption atomique (10), et elle est optiquement couplée à travers la deuxième fenêtre (5) à un rétroréflecteur affecté de sorte qu'un rayonnement d'exploration du spectromètre d'absorption atomique (10) qui est passé à travers la première fenêtre (5) dans la deuxième fenêtre (5) retourne à travers la deuxième fenêtre (5) dans le spectromètre d'absorption atomique (10) à travers la première fenêtre (5).
